# EUROPEAN PATENT SPECIFICATION

(11) **EP 2 883 055 B1**
(45) Date of publication and mention of the grant of the patent: **26.10.2016**
(21) Application number: 13765518.9
(22) Date of filing: 02.08.2013
(51) Int. Cl.: G01N 33/58, G01N 33/543, B82Y 15/00, B82Y 5/00

(54) **METHOD FOR THE PRODUCTION OF THERMOCHEMILUMINESCENT SILICA NANOPARTICLES AND THEIR USE AS MARKERS IN BIOANALYTIC METHODS**
VERFAHREN ZUR HERSTELLUNG VON THERMOCHEMILUMINESZENTEN KIESELSÄURENANOPARTIKELN UND IHRE VERWENDUNG ALS MARKER BEI BIOANALYTISCHEN VERFAHREN
PROCÉDÉ POUR LA FABRICATION DE NANOPARTICULES DE SILICE THERMOCHIMIOLUMINESCENTES ET LEUR UTILISATION COMME MARQUEURS DANS DES PROCÉDÉS BIOANALYTIQUES

(30) Priority: 10.08.2012 IT BO20120444
(43) Date of publication of application: 17.06.2015
(73) Proprietor: R.D. Pharmadvice S.r.l., 40124 Bologna (IT)
(72) Inventor: DI FUSCO, Massimo, I-40131 Bologna (IT); GUARDIGLI, Massimo, I-48019 Roncalceci (Ravenna) (IT); LOMBARDO, Marco, I-40141 Bologna (IT); MIRASOLI, Mara, I-40133 Bologna (IT); QUINTAVALLA, Arianna, I-40069 Zola Predosa (Bologna) (IT); RODA, Aldo, I-40123 Bologna (IT); TROMBINI, Claudio, I-40132 Bologna (IT)
(74) Representative: Biggi, Cristina
(86) International application number: PCT/IB2013/056340
(87) International publication number: WO 2014/024106

(56) References cited:
- WO-A2-2006/029302
- HUMMELEN J C ET AL: "Stable 1,2-dioxetanes as labels for thermochemiluminescent immunoassay", METHODS IN ENZYMOLOGY; [METHODS IN ENZYMOLOGY], ACADEMIC PRESS, US, vol. 133, 1 January 1986 (1986-01-01), pages 531-557, XP008160709, ISSN: 0076-6879, DOI: 10.1016/0076-6879(86)33088-X [retrieved on 2003-11-27]
- IMANISHI T ET AL: "Synthesis and Chemiluminescent Property of the Novel 1,2-Dioxetanes Containing an Acridane-10-acetate Moiety as the Luminophore and Trigger Unit", TETRAHEDRON LETTERS, PERGAMON, vol. 38, no. 5, 3 February 1997 (1997-02-03), pages 841-844, XP004188475, ISSN: 0040-4039, DOI: 10.1016/S0040-4039(96)02463-X
- SAYED M SALEH ET AL: "Novel multicolor fluorescently labeled silica nanoparticles for interface fluorescence resonance energy transfer to and from labeled avidin", ANALYTICAL AND BIOANALYTICAL CHEMISTRY, SPRINGER, BERLIN, DE, vol. 398, no. 4, 6 May 2010 (2010-05-06), pages 1615-1623, XP019839295, ISSN: 1618-2650
- Aldo Roda ET AL: "Dioxetane-Doped Silica Nanoparticles as Ultrasensitive Reagentless Thermochemiluminescent Labels for Bioanalytics", Analytical chemistry, 2 November 2012 (2012-11-02), pages 9913-9919, XP055056017, DOI: 10.1021/ac302306u| Retrieved from the Internet: URL:http://pubs.acs.org/doi/pdf/10.1021/ac 302306u [retrieved on 2013-03-11]

## Description

### Field of the invention

The present invention relates to the field of bioanalytical chemistry and provides thermochemiluminescent markers which can be used in the development of bioanalytical methods with thermochemiluminescent detection. In particular, the present invention provides silica nanoparticles containing a large number of thermochemiluminescent molecules, possibly in combination with other species capable of modifying and/or enhancing their emission properties, and functionalized on their surface with functional groups that enable them to be stably bonded to biospecific molecules, such as proteins, antibodies and nucleic acids.

### Background of the invention

Luminescence-based detection methods are particularly advantageous in the development of bioanalytical methods and biosensors and have numerous applications for both research and commercial purposes. Luminescence phenomena (production of photons by a chemical species) can be distinguished based on the type of stimulus that leads to the production of photons, which can be a physical one, such as the absorption of electromagnetic radiation, in the case of photoluminescence (fluorescence and phosphorescence); or else chemical. Luminescence phenomena of a chemical type can be further distinguished into: chemiluminescence, produced as a result of a chemical reaction triggered by mixing two or more reagents or catalyzed by an enzyme; bioluminescence, produced by enzymes or photoproteins present in living organisms or isolated from them; electrochemiluminescence (or electrogenerated chemiluminescence), in which the reagent species are generated as a result of an electrochemical reaction triggered by application of a potential; finally, thermochemiluminescence, in which the trigger that starts the chemiluminescent reaction is of a thermal type.

Fluorescence-based detection techniques are widely used in bioanalytical chemistry, but, despite being characterized by high emission intensities, they show limitations due to phenomena of autofluorescence (typical of the biomolecules present in biological specimens) and of an instrumental type (e.g. scattering of the excitation light), relatively high detection limits due to the low extinction coefficient or low quantum efficiency of many organic fluorophores, and difficulty in obtaining quantitative information. Moreover, the implementation of fluorescence-based detection techniques in compact portable analytical instruments is made difficult by the need to include a photoexcitation source and systems for selecting both the excitation and emission wavelength.

Chemiluminescence-based detection techniques, though less widely disseminated, offer numerous advantages, since they are characterized by high detectability (the base signal is extremely low being essentially due to instrument noise) and instrumental simplicity (excitation lamps and filters are not required). Chemiluminescence is thus particularly well suited to the development of ultrasensitive, miniaturized analytical devices. Its main disadvantage is that one or more reagents need to be added in order to start the emission of photons and it requires a microfluidic system to deliver these reagents. Moreover, in many cases the reagent consists of two components which must be rapidly and efficiently mixed only at the time of measuring, further complicating the design of the system microfluidics.

Electrochemiluminescence has been successfully applied in the development of automated diagnostic systems and in recent years has also had numerous applications in the development of miniaturized instruments, with analytical performances similar to those of chemiluminescence. In this case as well, the reaction requires the application of specific reagents at the time of measuring, with a consequent complication of the microfluidic system.

Thermochemiluminescence, i.e. the emission of light deriving from the decomposition of molecular species caused by heating, was proposed in the 1980s as a detection technique for bioanalytical methods. Although it was abandoned after several pioneering applications in immunological analyses, this luminescence technique potentially makes it possible to overcome the limitations posed by chemiluminescence and electrochemiluminescence in the development of miniaturized analytical instruments, since it does not require the addition of reagents for measuring, simply the application of a thermal shock. Thermochemiluminescence should not be confused with the thermoluminescence, which is an emission of light deriving from physical (and not chemical) processes triggered by heating (e.g. the recombination of free electrons and electron holes) and which occurs in crystalline materials.

The thermal and chemical stability of thermochemiluminescent markers enables them to be stored for long periods and makes them compatible with different experimental conditions (wide pH range, solvents, biological molecules, etc. Thermochemiluminescent molecules can moreover be synthesized in a cost-effective manner, resulting in products characterized by a high degree of purity and specific activity.

Many chemiluminescent reactions exhibit intermediate reactions with the structure of 1,2-dioxetane. In the quest for molecules that can be used as thermochemiluminescent markers, therefore, numerous molecules containing the 1,2-dioxetane group have been synthesized; for the most part, they are unstable at room temperature, and thus have little application in bioanalytical chemistry. However, some molecules have shown to be sufficiently stable for a potential practical application.

For example, Wieringa et al. *(*Wieringa, J.H. et al. Tetrahedron Lett. 1972 2:169-172) describe the synthesis of the compound adamantylidene adamantane 1,2-dioxetane, which shows high stability and a half-life at 25 °C calculated to be 10⁴ years. Imanishi et al. (Imanishi, T. et al. Tetrahedron Lett. 1997 38:841-844*)* report the synthesis of compounds with the structure of 3'-spiro[9-acridane]-4'-spiro[adamantyl]-1,2-dioxetane with different substituents in position 10. The authors propose using these molecules as chemiluminescent tracers by promoting the emission of photons by hydrolysis in an alkaline environment. No mention is made of the possibility of using these molecules as thermochemiluminescent markers.

1,2-dioxetanes decompose on being heated, producing carbonylic fragments. By virtue of the high tension energy of the parent molecule, a fraction of one of the two fragments is produced in an excited singlet or triplet state. These excited fragments can emit radiation, both by a direct mechanism (the excited fragment decays to the fundamental state, emitting a photon) and an indirect mechanism (the excited fragment transfers its energy to a fluorescent energy acceptor, bringing it to an electronically excited state; the excited fluorescent acceptor in turn decays to the fundamental state, emitting a photon).

The possibility of having emission through one or the other of the two mechanisms and the temperature at which this process actually occurs depend on the nature of the 1,2-dioxetane. If the carbolic fragments produced by the decomposition reaction are only weakly fluorescent (as, for example, in the case of adamantylidene adamantane 1,2-dioxetane), it is possible to obtain a sufficiently intense emission only in the presence of a fluorescent acceptor, whereas if one of the two carbonylic fragments is an efficient fluorophore, emission can also be obtained in the absence of an acceptor.

It is however reasonable to expect that, in this case as well, the emission will be more intense in the presence of a fluorescent acceptor, since it could have a better emission yield or intercept any non-excited non-fluorescent carbonyl fragments.

A thermochemiluminescent reaction is a first-order process; thus the reaction kinetics is independent from the concentration of the marker in the specimen and depends on temperature alone. Therefore, the analytical signal can be corrected by eliminating nonspecific signals by means of suitable algorithms.

Hummelen et al. (Hummelen, J.C. et al. Meth. Enzymol. 1986 133:531-557) report the synthesis of thermochemiluminescent markers derived from the molecule adamantylidene adamantane 1,2-dioxetane with the introduction of reactive groups (e.g. maleimide or N-hydroxysuccinimide ester). The authors illustrate the synthesis schemes and procedures for bioconjugating the marker with free reactive groups (e.g. thiols or amines) present on proteins or antibodies.

Hummelen et al. (*ibid*) describe an immunological method with thermochemiluminescent detection for the determination of human chorionic gonadotropin (hCG) using Kapton discs functionalized with a capture antibody and a second anti-hCG antibody marked with a derivative of adamantylidene adamantane 1,2-dioxetane. The direct conjugation of antibodies with thermochemiluminescent markers is made difficult by their low water solubility. During bioconjugation it is therefore necessary to use an organic cosolvent (1,4-dioxane, tetrahydrofuran, dimethylformamide) in order to increase the conjugation yield. However, in order to avoid denaturing the antibodies, the percentage of such cosolvents must be relatively low (generally no higher than 5% v/v), thus limiting the reaction yield. Moreover, the conjugation of a large number of markers on an antibody molecule would result in a significant loss of its immunological activity due to steric hindrance and the possibility of bonding marker molecules in positions near or corresponding to the antibody bonding site. It is thus more advantageous to synthesize a bioconjugate using an inert carrier protein (e.g. BSA), which is loaded with a high number of markers and subsequently conjugated to the antibody.

Furthermore, the direct thermochemiluminescence of the derivates of 1,2-dioxetanes generally results in the emission of very weak luminescence (with an efficiency of about 1% relative to the chemiluminescence of the luminol) due to the low fluorescence efficiency of the reaction products (ketones, esters, aldehydes). Hummelen et al. (*ibid*) describe the synthesis of a thermostable fluorescent acceptor 2-[O-(N-succinimidyl)carboxypropyl]-9,10-diphenylanthracene (SCP-DPA) which enables the signal of adamantylidene adamantane 1,2-dioxetane to be amplified. Hummelen et al. (*ibid*) describe the development of a non-competitive immunological method for determining the carcinoembryonic antigen (CEA) based on the use of a monoclonal anti-CEA capture antibody immobilized on Kapton discs and a polyclonal anti-CEA detection antibody marked with a derivative of adamantylidene adamantane 1,2-dioxetane and molecules of SCP-DPA.

Hummelen et al. (*ibid*) describe an immunological method with thermochemiluminescent detection (Fluorescence Amplified Thermochemiluminescence IMmunoAssay, FATIMA) based on the use of markers consisting of a BSA molecule functionalized by means of covalent bonds with a relatively high number of adamantylidene adamantane 1,2-dioxetane derivatives and the fluorescent acceptor SCP-DPA.

Hummelen et al. (*ibid*) describe the development of a non-competitive ("sandwich"-type) immunological method for determining human immunoglobulin G (hlgG) based on the use of an anti-hlgG capture antibody immobilized on Kapton discs and an anti-hlgG detection antibody marked with a BSA molecule previously marked with a adamantylidene adamantane 1,2-dioxetane derivative and molecules of SCP-DPA, according to the FATIMA principle.

The FATIMA method requires a relatively complex instrumental set-up and the use of heat-resistant materials (Kapton or Teflon) to construct an analytical medium for the immunological reaction and subsequent thermochemiluminescent reaction, since the thermoluminescent marker decomposition temperature necessary in order to reach the required detectability is 200-250 °C. The high temperature precludes detection in an aqueous solution and the use of commercially available instruments for measuring bioluminescence and chemiluminescence.

The FATIMA method has been applied in the development of several immunological methods, whereas no applications related to nucleic acid hybridization or microscopic image analysis have ever been reported. After a number of applications described in the literature, the FATIMA method was abandoned, as it offered scant detectability and entailed greater instrumental complexity than methods based on enzymatic or fluorescent markers.

As reported by Hummelen et al. (*ibid*)*,* one of the main disadvantages of the FATIMA method is the loss of linearity and reproducibility of the thermochemiluminescent signal due to phenomena of thermochemical degradation of the spacer which acts like a bridge between the marker and the carrier protein. This phenomenon causes a partial evaporation of the thermochemical marker prior to the emission of thermochemiluminescence.

They propose complexing the thermochemiluminescent marker with γ-cyclodextrin or incorporating it in cholesterol/phosphatidylcholine liposomes. Both of these strategies inhibit the evaporation of the marker, making it possible to obtain excellent signal linearity and reproducibility, and shield the marker from interfering agents present in the biological matrix of the specimen, but they have never been applied in the development of bioanalytical methods.

A significant reduction in the trigger temperature of the thermochemiluminescent marker would allow these types of problems to be avoided and would simplify the design of instruments for the measurement of thermochemiluminescence. Furthermore, it has never been proposed to use other types of media besides proteins to carry out marking with thermochemiluminescent molecules. The use of media of a different nature, which could permit the incorporation of a high number of thermochemiluminescent molecules for marking, would significantly improve the analytical performances of thermochemiluminescent detection.

Nanomaterials are materials characterized by a length ranging between 1 and 100 nm in at least one of their dimensions. Nanoparticles can be obtained from various organic and inorganic materials. Silica nanoparticles containing a high number of luminescent molecules are broadly used as markers in the development of bioanalytical methods, as they offer a high signal amplification factor and protect the fluorescent molecule against contact with possible chemical interferents. Such nanoparticles have been used to detect pathogens, proteins and nucleic acids in a wide variety of biological specimens *(*Zhong, W.W. Anal. Bioanal. Chem. 2009 394:47-59*).* Moreover, silica nanoparticles can be easily functionalized on their surface for bonding with biomolecules and exhibit high biocompatibility and chemical stability.

Silica nanoparticles incorporating luminescent markers are generally produced with microemulsion-based methods or else using the Stöber method. The nanoparticles must be subsequently functionalized by introducing reactive groups that enable bioconjugation to proteins or DNA molecules through the formation of covalent bonds with free amino or carboxyl groups present on such molecules *(*An Y, et al. J. Colloids Interface Sci. 2007 311:507-513*;* Hermanson, G.T. (ed.) Bioconjugate Techniques, 2nd Edition, Academic Press, 2008 p. 574*).*

Numerous authors have reported the production of luminescent silica nanoparticles which incorporate fluorescent (McDonagh, C. et al. Nanomedicine 2009 4:645-656*),* chemiluminescent and electrochemiluminescent (Giokas, D.L. et al. Trends Anal. Chem. 2010 29:1113-1126*)* molecules or enzymes *(*Ansari, S.A. et al. Biotech. Adv. 2012 30:512-523)*.*

There are also a large number of patents relating to the production and application of fluorescent nanoparticles or nanoparticles containing fluorescent molecules.

Patent EP2364840(A1) - Fluorescent silica-based nanoparticles - describes the preparation of fluorescent monodispersed silica nanoparticles having a diameter of less than 30 nm and functionalized with covalently attached organic fluorophores. The nanoparticles have an architecture (core-shell) which makes it possible to obtain a high fluorescence quantum efficiency compared to the free fluorophore and to control the photophysical properties of the fluorophore.

Patent KR20120005749(A) - Fluorescence hollow silica nanoparticle and preparation method thereof - describes the preparation of fluorescent silica nanoparticles with a hollow core with a diameter of 1-100 nm and a silica shell containing the fluorophore. Patent KR20090120994(A) - Fullerene-silica nanoparticles having improved luminescence, preparation method thereof and use thereof - describes the preparation of silica nanoparticles functionalized with fullerene.

Patent JP2009265067(A) - Silica nanoparticle labeled with molecular recognition fluorescence - describes the preparation of silica nanoparticles functionalized on the surface with a fluorophore and a specific molecular recognition polymer.

Patent WO2011109214(A2) - Near-IR indocyanine green doped multimodal silica nanoparticles and methods for making the same - describes the preparation of nanoparticles with a core-shell architecture comprising a core which contains the indocyanine green marker and a silica shell. The nanoparticles are used as near-infrared fluorescence markers and display high photostability. The nanoparticles described can be modified so as to be used as markers for in vivo and in vitro imaging, or as photodynamic therapeutic agents.

Thermochemiluminescent markers are particularly suitable for being incorporated into nanoparticles, since they are not subject to phenomena of self-quenching or absorption of emitted radiation as is typical of many luminescent molecules. Therefore, it is possible to incorporate a large number of thermochemiluminescent markers in nanostructures without observing any decrease in their specific activity. However, no nanoparticles containing or functionalized with thermochemiluminescent markers have been described to date.

### Summary of the invention

The present invention provides thermochemiluminescent silica nanoparticles that can be employed as markers for the development of bioanalytical methods, such as immunological and genetic methods, with thermochemiluminescent detection.

The nanoparticles have an almost spherical shape and nanometric dimensions which may vary according to the synthesis conditions (for example, the nanoparticles best suited for use as markers for biospecific molecules have average diameters of around 50 - 150 nm).

The nanoparticles contain within them a plurality of thermochemiluminescent molecules, preferably homogeneously dispersed in the silica matrix. The thermochemiluminescent molecules are contained in the nanoparticles in large number and they are responsible for the emission of light by the nanoparticle upon exposure to heat. The nanoparticle can preferably host a large number of thermochemiluminescent molecules, comprised from 1000 to 10000 per single nanoparticle. The number of thermochemiluminescent molecules contained in a single nanoparticle makes it possible to obtain a high signal amplification compared to what can be obtained with a single thermochemiluminescent molecule; hence the detectability of the nanoparticles in a measurement of thermochemiluminescence is much higher.

In particular, the invention relates to a thermochemiluminescent silica nanoparticle comprising a plurality of molecules of 1,2-dioxetane having the general formula (I): 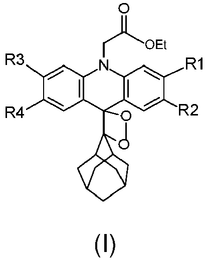 wherein R₁, R₂, R₃ and R₄, identical to or different from each other, are selected from among: -Me, -Et, -Pr, -iPr, -OMe, -OEt, -F, -Cl, -NH₂, -OH, -NO₂; or else R₁ and R₂, together with the carbon atom to which they are bonded, form an aromatic ring of 6 carbon atoms, and/or R₃ and R₄, together with the carbon atom to which they are bonded, form an aromatic ring of 6 carbon atoms.

Preferably R₁, R₂, R₃ and R₄ are selected from among: -Me, -Et, -Pr and -iPr.

In an alternative embodiment of the invention, the thermochemiluminescent silica nanoparticle comprises a plurality of molecules of 1,2-dioxetane having the general formula **(II)**: 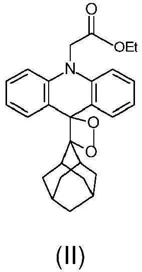 whose chemical name is ethyl-dispiro[acridine-9(10*H*),3'-[1,2]dioxetane-4',2"-tricyclo[3.3.1.1^{3,7}]decane]-10-acetate.

The nanoparticles can contain additional chemical species which have the function of modifying and/or improving the emission properties, e.g. fluorescent molecules capable of increasing the emission efficiency of the thermochemiluminescent reaction and/or of modifying the emission wavelength via a process of energy transfer from the excited products obtained from the decomposition of the thermochemiluminescent molecule. In particular, dipyridamole (hereafter indicated as DP), fluorescein, rhodamine and aromatic polycyclic hydrocarbons such as, for example, substituted anthracenes are considered fluorescent molecules.

The nanoparticles emit thermochemiluminescent radiation upon being heated at relatively low temperatures. In particular, in the nanoparticles of the invention, thermochemiluminescence occurs below 100 °C; hence the reaction can also occur in an aqueous environment.

The nanoparticles possess surface reactive groups, which are introduced during synthesis or at a subsequent stage and permit their stable bonding to biospecific probes (proteins, antibodies, oligonucleotides, PNA and aptamers). In particular, the reactive groups can be amines, carboxyls, thiols, etc. and can incorporate spacer groups of varying nature and length to facilitate the conjugation reaction.

The nanoparticles can thus be used as thermochemiluminescent markers for the development of bioanalytical procedures characterized by high sensitivity and simplicity of execution, and suitable also for use in miniaturized portable systems. The possible bioanalytical procedures include reactions of an immunological type and nucleic acid hybridization (e.g. immunometric analyses, nucleic acid hybridization analyses, immunocytochemistry, flow cytometry, DNA or protein microarray analyses, etc.) in their various analysis formats (microplates, microfluidic systems, microarrays, lateral flow immunoassay, etc.).

The nanoparticles can be advantageously implemented in bioanalytical procedures for the development of techniques of analysis that do not require any external source of radiation (as in fluorescence) or addition of chemical reagents (as in biochemiluminescence).

Another particular feature of thermochemiluminescence, which distinguishes it from other chemical luminescence phenomena (e.g. biochemiluminescence) is that since there is no need for reagents, but only for simple heating, the emission can also be obtained in a dry environment, in the absence of any solvent or liquid medium.

### Detailed description of the invention

The present invention will now be described in detail, also by means of diagrams, wherein:
Figure 1 shows the basic scheme used to obtain the thermochemiluminescent 1,2-dioxetanes **(I)** and **(II)** employed in the synthesis of the thermochemiluminescent silica nanoparticles;
Figure 2 shows (A) a TEM image of the silica nanoparticles doped with one of the thermochemiluminescent 1,2-dioxetanes **(II)** and DP, (B) the kinetics of the thermochemiluminescent emission obtained from the aforesaid nanoparticles by heating them to 90 °C and (C) an image of the thermochemiluminescent signal obtained from the silica nanoparticles after heating to 90 °C;
Figures 3 and 4 show diagrams of the reactions used to couple the thermochemiluminescent silica nanoparticles to IgG by exploiting the surface amino groups of the nanoparticles.
Figure 5 shows examples of calibration curves obtained in two model assays, one based on immunological reactions (determination of ovalbumin) and one based on nucleic acid hybridization reactions (determination of Parvovirus B19 DNA), which use the thermochemiluminescent nanoparticles as markers.

The thermochemiluminescent silica nanoparticles of the present invention contain within them the thermochemiluminescent 1,2-dioxetanes **(I)** and/or **(II),** characterized by the presence of an adamantyl group (which performs a molecule stabilization function) and of a fluorescent group acridine *N*-ethyl acetate (which is produced in an electronically excited state and is responsible for the emission of thermochemiluminescence upon heating of the nanoparticle). These thermochemiluminescent 1,2-dioxetanes decompose with the emission of radiation at a particularly low decomposition temperature (50-70 °C).

It is understood that the present invention relates to thermochemiluminescent silica nanoparticles containing all the 1,2-dioxetanes of formula (I), optionally in a mixture with the 1,2-dioxetane of formula (II), on their own or in the presence of fluorescent acceptors.

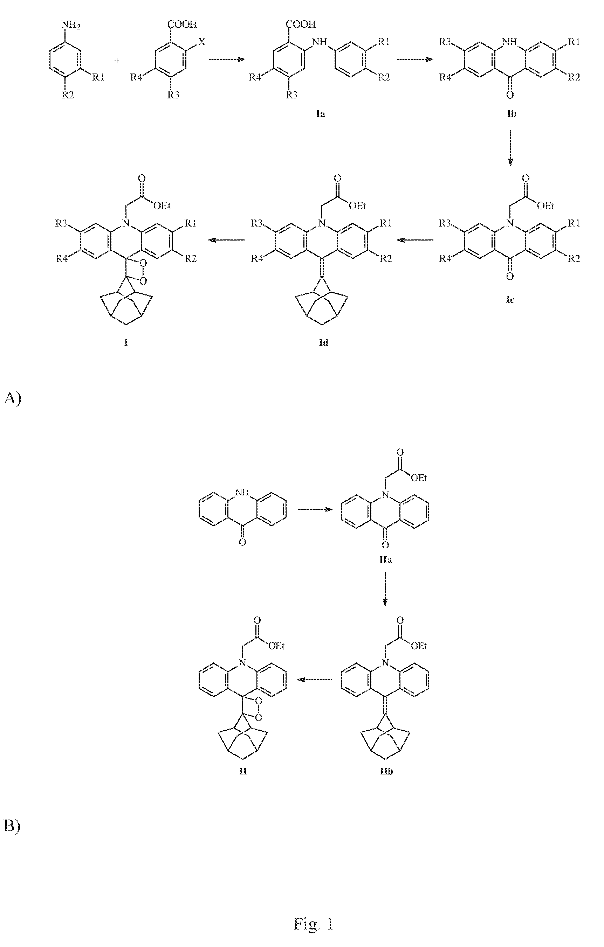

The thermochemiluminescent 1,2-dioxetanes **(I)** and **(II)** were synthesized as schematically illustrated in Figure 1 and described hereunder, by adapting procedures described in the literature (Dalcanale, E. et al. J. Org. Chem. 1986 51:567-569*;* Marzena, O.-Z. et al. Synthesis 2007 21:3345-3356*;* Stankiewicz-Drogo, A. et al. J. Med. Chem. 2010 53:3117-3126*;* David-Cordonnier, M.-H. et al. Eur. J. Chem. Med. 2007 42:752-771*;* Imanishi. T. et al. ibid*;* Schaap, A.P. et al. J. Am. Chem. Soc. 1975 97:3741-3745)*.*

The materials used in the synthesis are: variously substituted anilines, 2-aminonaphthalene, variously substituted 2-bromobenzoic acids, variously substituted 2-iodobenzoic acids, copper powder (≥99.5%), potassium carbonate (99%), 1-pentanol, phosphorus oxychloride (V) (99%), sodium hydride (97%), 9(10*H*)-acridone (99%), ethyl-2-bromoacetate (98%), tetrabutylammonium iodide (98%), titanium chloride (III) complexed with tetrahydrofuran (complex 1:3, 97%), lithium aluminium hydride (95%), 2-adamantanone (99%), polystyrene-supported Rose Bengal, and Methylene Blue. All of the solvents used in the syntheses were previously anhydrified.

Compounds **Ia** were synthesized as follows: a mixture of variously substituted anilines or 2-aminonaphthalene (6.5 mmol) and variously substituted 2-bromobenzoic acids or 2-iodobenzoic acids (4.7 mmol) with K₂CO₃ (6.5 mmol) and copper powder (0.9 mmol) in 1-pentanol (5.0 mL) was heated under reflux for 3 hours. The solvent was vacuum evaporated, the solid residue was dissolved in hot water (100 mL) and then filtered through Celite. The Celite was washed with water (300 mL) and the filtrate was acidified to pH 6 with concentrated HCl. The precipitate was isolated by filtration and washed with water. The resulting solid was recrystallized by CHCl₃. Compounds **Ib** were synthesized as follows: compounds **Ia** (4.0 mmol) were dissolved in CH₃CN (9 mL) and heated under reflux. Phosphorus oxychloride (V) (8.8 mmol) was added over a period of 1 hour. The solution was heated under reflux for another 2 hours and subsequently cooled to 10-15 °C. After that, H₂O (5 mL) was added and the reaction was heated under reflux for 2.5 hours. The final suspension was cooled to 10 °C and filtered. The resulting solid was washed with H₂O and CH₃CN and then vacuum dried.

Compounds **Ic** and **IIa** were synthesized as follows: NaH (1.2 mmol) (97%) was suspended in 2 ml of DMF, a suspension of compounds **Ib** and 9(10*H*)-acridone (0.9 mmol) in 4 ml of DMF was added and the mixture was magnetically stirred for 30 minutes at room temperature. The mixture was cooled to 0°C, ethyl-2-bromoacetate (1.4 mmol) and tetrabutylammonium iodide (0.01 mmol) were added and the mixture was magnetically stirred for another 24 hours at room temperature. The raw reaction product was poured into cold water, the precipitate was filtered, vacuum dried and purified by flash chromatography on silica gel (dichloromethane/ethyl acetate = 9/1). Compounds **Id** and **IIb** were synthesized as follows: LiAlH₄ (1.08 mmol) was added to a suspension of TiCl₃·3THF (1.08 mmol) in THF (1.5 mL) at 0 °C and the resulting suspension was magnetically stirred for 10 minutes. After triethylamine (1.08 mmol) was added at room temperature, the reaction was left under reflux for 1 hour. A solution of compounds **Ic** and **IIa** (0.36 mmol) and 2-adamantanone (0.36 mmol) in THF (3.0 mL) was added drop by drop over a period of 30 minutes. After that, the reaction was refluxed for 17 hours, cooled at room temperature and filtered through silica. The silica was washed with dichloromethane and the filtrate was dried. The raw product was purified by flash chromatography on silica gel (dichloromethane/hexane=1/1).

Compounds **I** and **II** were synthesized as follows: compounds **Id and IIb** (0.07 mmol) were dissolved in CH₂Cl₂ (6 ml), 0.05 g of polymer-supported Rose Bengal or Methylene Blue was added and the suspension was cooled to 0°C. The solution was bubbled with oxygen, magnetically stirred and irradiated with a halogen lamp (400 W) fitted with a filter capable of cutting out ultraviolet light (transmission at 400 nm = 0.5%) for the entire duration of the reaction. The raw reaction product was purified by activated carbon filtration.

All of the synthesized compounds were characterized by recording ¹H and ¹³C NMR spectra and mass spectra.

The thermochemiluminescent silica nanoparticles of the invention were synthesized using a microemulsion procedure with controlled hydrolysis of triethoxyvinylsilane in the nonpolar core of detergent-stabilized micelles of n-butanol in water. The procedure for synthesizing the nanoparticles enables the thermochemiluminescent 1,2-dioxetane and the fluorescent acceptor, where present, to be incorporated during the synthesis itself. During synthesis, the nanoparticles acquire the surface functionalization with amino groups used in the procedures for conjugating the nanoparticles to the biospecific probes.

The materials used in the synthesis of the thermochemiluminescent nanoparticles are: dioctyl sulfosuccinate sodium salt (AOT), n-butanol (anhydrous, >99%), N,N-dimethylformamide (DMF, anhydrous, >99%), triethoxyvinylsilane (TEOVS, 97%), 3-aminopropyltriethoxysilane (APTES, 99%), dipyridamole (DP, >98%) and absolute ethanol, all used without further purification. Deionized water was also used (>18 MΩ·cm).

### Example 1 - Synthesis of compounds I having R₁ = R₂ = R₃ = R₄ = -Me.

The synthesis was carried out as previously described. The above-mentioned compounds were obtained with yields of 23-84%.

### Example 2 - Synthesis of compound II.

The synthesis was carried out as previously described. Compound **II** was obtained with a yield 64% *(*Roda, A. et al. Anal. Chem. 2012 84:9913-9919)*.*

### Example 3 - Synthesis of nanoparticles doped with compounds I and II.

0.44 g of AOT and 800 µl of 1-butanol were dissolved in 20 ml of deionized water and the solution was vigorously shaken. 30 µl of compounds **I** and **II** (30 mmol l⁻¹) in DMF was added to the solution and the mixture was magnetically stirred. After that, triethoxyvinylsilane (200 µl) was added and the resulting solution was stirred for about 1 hour. When a clear solution was obtained, 10 µl of 3-aminopropyltriethoxysilane was added and the mixture was stirred for another 20 hours. The entire reaction was carried out at room temperature. Finally, the nanoparticles were separated by centrifugation, washed several times with deionized water and stored dispersed in water at -20 °C (*Roda, A. et al. ibid*)*.*

The use of compounds **I** and **II** dissolved in DMF solves two fundamental problems in the synthesis and stability of the doped nanoparticles of the invention. The compound used for doping the nanoparticles of the invention is not water soluble, but it was seen that if a solution of **I** and **II** in DMF is added to the microemulsion, the 1,2-dioxetane migrates into the micelles to form the doped nanoparticles. The use of n-butanol as a co-surfactant has no negative effects on the formation of the microemulsion.

### Example 4 - Synthesis of nanoparticles doped with compounds I and II and DP.

The synthesis of nanoparticles was carried out according to the procedure described above, but 30 µl of compounds **I** and **II** and DP (respectively 30 and 60 mmol l⁻¹) in DMF was added to the solution.

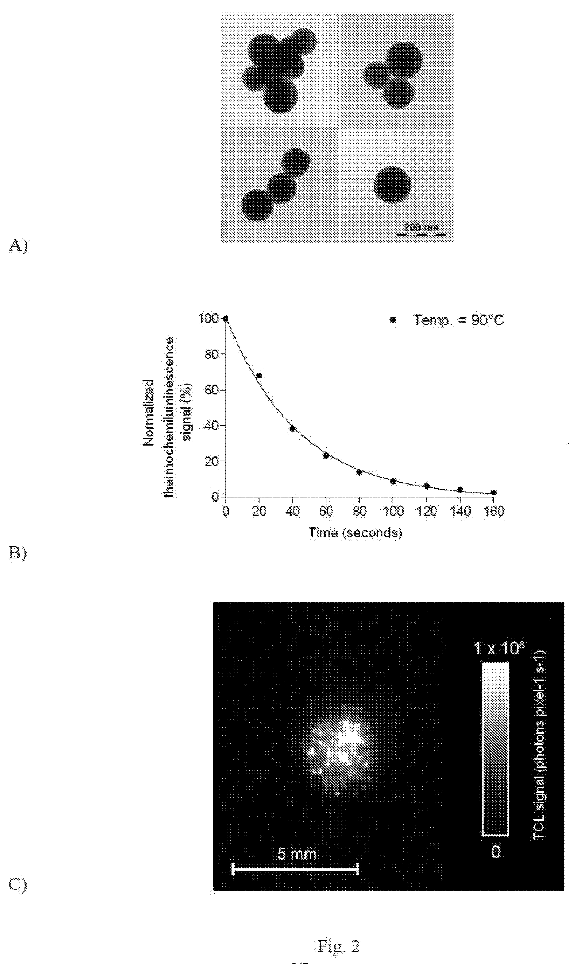

The doped nanoparticles were characterized by using Transmission Electron Microscopy (TEM). The mean diameter of the doped silica nanoparticles was 110 ± 10 nm (Figure 2A).

The degree of incorporation of compounds I and **II** and DP into the silica nanoparticles was evaluated by spectrophotometrically measuring the residual compounds in the wash water of the synthesis process. The incorporation yields obtained were 43% and 93%, respectively for compounds **I** and **II** and DP. It was estimated that every nanoparticle contained about 10⁴ molecules of compounds **I** and **II** and 4×10⁴ molecules of DP.

The thermochemiluminescence characteristics of the nanoparticles were determined by luminescence imaging using an LB-980 Night Owl luminograph (Berthold Technologies). The measurements were made by heating the samples - deposited on a glass surface - using a flat heating element. The surface temperature was monitored with a thermocouple consisting of copper/costantan.

The thermochemiluminescence emission of the silica nanoparticles doped with compounds **I** and **II,** both in the absence and presence of the fluorescent acceptor DP, takes place at temperatures that are relatively low (50-70 °C) and much lower than those of known 1,2-dioxetanes, since they have TCL fragmentation activation energies comprised from 16 to 30 kcal mol⁻¹. At a temperature of 90 °C, the thermochemiluminescent emission lifetime of the nanoparticles doped with **I** and **II** and DP is 20-30 seconds, as shown in Figure 2B. The kinetics of the thermochemiluminescent emission enables the doped nanoparticles to be used as markers for biospecific probes in bioanalytical methods based on thermochemiluminescence measurements, in which the detection stage requires heating at temperatures below 100 °C (so analyses can also be performed in an aqueous environment) and the measurement can be made in just a few minutes while collecting all of the radiation emitted by nanoparticles.

Thanks to the large number of 1,2-dioxetane molecules contained in a single nanoparticle, their detectability is much greater (by 3-4 orders of magnitude) than that of a single molecule of thermochemiluminescent 1,2-dioxetane. The detection limit obtained with thermochemiluminescence imaging measurements for the nanoparticles doped with compounds **I** and **II** and DP is comprised from 1 × 10⁻¹⁶ to 1 × 10⁻¹⁷ moles mm⁻² versus the 2 × 10⁻¹³ to 2 × 10⁻¹⁴ moles mm⁻² obtained for the thermochemiluminescent molecules **I** and **II** under the same conditions. The doped nanoparticles will thus enable the development of bioanalytical methods with thermochemiluminescent detection that are very sensitive thanks to their high detectability.

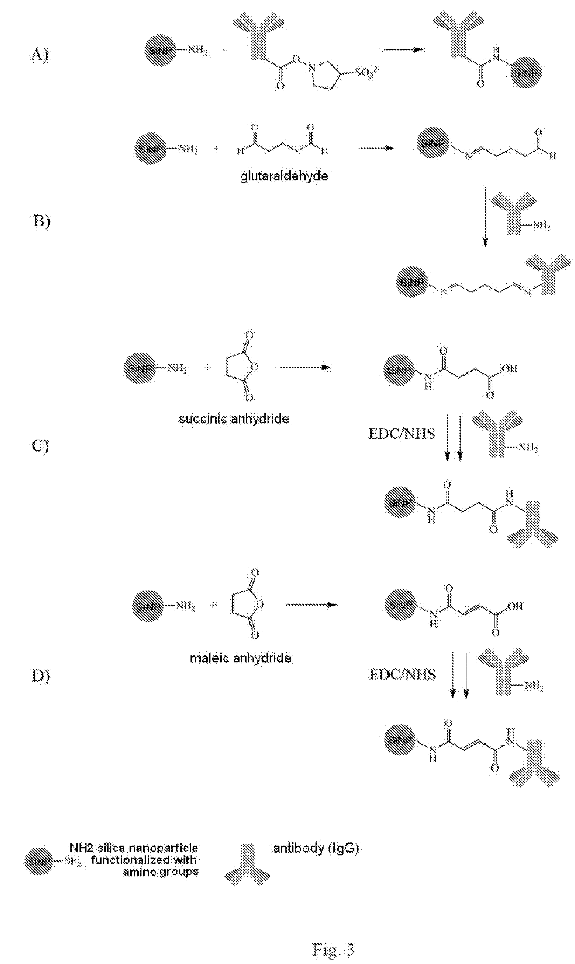

For the purpose of developing bioanalytical methods, the nanoparticles were conjugated to antibodies (immunoglobulin IgG) using different procedures, described in the examples below and schematically illustrated in Figure 3; these procedures exploit the surface amino groups introduced into the nanoparticles during synthesis. The materials used to conjugate the thermochemiluminescent nanoparticles to antibodies were: *N*-(3-dimethylaminopropyl)-*N'*-ethylcarbodiimide hydrochloride (EDC, >99%), *N*-hydroxysuccinimide (NHS, 98%), *N*-hydroxysulfosuccinimide sodium salt (sulfo-NHS, >98.5%), glutaraldehyde in solution (GA, 50% in water), succinic anhydride (97%), maleic anhydride (95%), 4-(*N-*maleimidomethyl)cyclohexane-1-carboxylic acid 3-sulfo-*N*-hydroxysuccinimide ester sodium salt (sulfo-SMCC), 2-iminothiolane hydrochloride (>98%), S-acetylthioglycolic acid *N*-hydroxysuccinimide ester (SATA, >95%), 2-(*N*-morpholino) ethanesulfonic acid (MES, >99%), 2-mercaptoethanol (>99%), ethanolamine (>99%), ethylenediaminetetraacetic acid (EDTA, >99%), 2-mercaptoethylamine (2-MEA, 95%), hydroxylamine hydrochloride (99%), sodium borohydride (>96%), sodium chloride (>99.5%), sodium carbonate (>99%), monobasic sodium phosphate (>99%), and dibasic sodium phosphate (>99%), all used without further purification.

### Example 1 (Figure 3A) - conjugation with N-hydroxysulfosuccinimide.

0.1 mg of antibody was dissolved in 100 µl of 0.05 M MES and 0.5 M NaCl buffer, pH 6 (final concentration 1 mg ml⁻¹). 50 µl of 8 mM EDC and 50 µl of 20 mM sulfo-NHS in Milli-Q water were added to this solution (final concentrations 2 mM of EDC and 5 mM of sulfo-NHS). The resulting mixture was made to react under stirring for 15 minutes at room temperature. Then 3 µl of 2-mercaptoethanol was added (final concentration 20 mM) and the solution was stirred for 10 minutes at room temperature. After that, 500 µl of doped nanoparticles (1 mg ml⁻¹ in PBS buffer, pH 7.5) was added and the mixture was made to react for 2 hours at room temperature. Finally, the nanoparticles conjugated with the antibody were collected by centrifugation and washed several times with Milli-Q water.

### Example 2 (Figure 3B) - conjugation with glutaraldehyde.

4 mg of doped nanoparticles were dispersed in 400 µl of 0.1 M PBS and 0.15 M NaCl buffer, pH 6.8 (final concentration 10 mg ml⁻¹). 10 µl of 50% glutaraldehyde in water was added to this solution (final concentration 1.25%). The resulting solution was made to react under stirring overnight at room temperature. The nanoparticles were collected by centrifugation, washed several times with Milli-Q water and re-dispersed in 100 µl of 0.5 M carbonate buffer, pH 9.5. After that, 1 mg of antibody was added (final concentration 1 mg ml⁻¹, ratio by weight nanoparticles:antibody = 4:1) and the mixture was made to react overnight at 4 °C. To reduce the resulting Schiff base and excess aldehyde, 1 mg of sodium borohydride was added (final concentration 10 mg ml⁻¹) and the mixture was made to react for 1 hour at 4 °C. Finally, the nanoparticles conjugated with the antibody were collected by centrifugation and washed several times with Milli-Q water.

### Example 3 (Figure 3C, - conjugation with succinic anhydride.

1 mg of doped nanoparticles was dispersed in 1 ml of 0.1 M PBS buffer, pH 7.0 (final concentration 1 mg ml⁻¹), 1 mg of succinic anhydride was added (final concentration 1 mg ml⁻¹) and the mixture was made to react for 2 hours at room temperature. The nanoparticles were then collected by centrifugation, washed several times with Milli-Q water and re-dispersed in 100 µl of 0.05 M MES and 0.5 M NaCl buffer, pH 6 (final concentration 1 mg ml⁻¹). 50 µl of 8 mM EDC and 50 µl of 20 mM sulfo-NHS, both in Milli-Q water, were added to this solution (final concentrations 2 mM of EDC and 5 mM of sulfo-NHS). The resulting solution was made to react under stirring for 15 minutes at room temperature. Then 3 µl of 2-mercaptoethanol was added (final concentration 20 mM) and the solution was stirred for 10 minutes at room temperature. After that, 500 µl of 1 mg ml⁻¹ antibody in 0.1 M PBS buffer, pH 7.5, was added and the mixture was made to react for 2 hours at room temperature. Finally, the nanoparticles conjugated with the antibody were collected by centrifugation and washed several times with Milli-Q water.

### Example 4 (Figure 3D) - conjugation with maleic anhydride.

The procedure for conjugating the nanoparticles to the antibody using maleic anhydride is substantially identical to the one described for succinic anhydride (Example 3), except for the pH during the first reaction, which must be maintained alkaline (pH 8-9) to prevent undesirable deacylation.

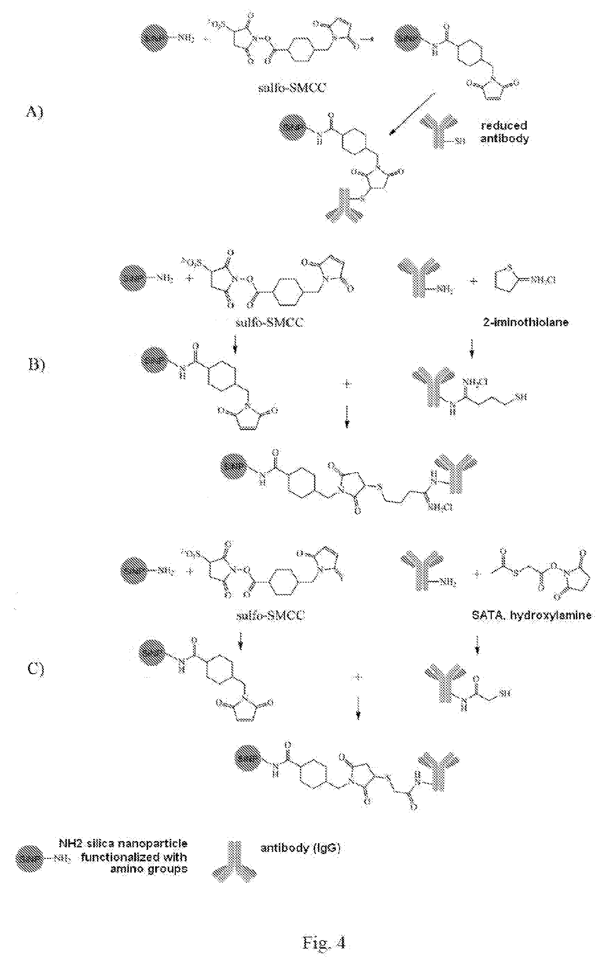

### Example 5 (Figure 4A) - conjugation by activation of the nanoparticles with sulfo-SMCC and reduction of the antibody with 2-mercaptoethylamine.

This method is divided into pre-activation of the doped nanoparticles, reduction of the antibody and conjugation in three separate reactions. 1) Activation of doped nanoparticles: 3 mg of nanoparticles was dispersed in 100 µl of 0.1 M PBS and 0.15 M NaCl buffer, pH 7.2 (final concentration 30 mg ml⁻¹), 6 mg of sulfo-SMCC was added and the mixture was made to react for 30 minutes at room temperature. The nanoparticles modified with sulfo-SMCC were collected by centrifugation, washed several times with Milli-Q water and re-dispersed in 300 µl of 0.1 M PBS and 0.15 M NaCl buffer, pH 7.2 (final concentration 10 mg ml⁻¹). 2) Reduction of the antibody: 1 mg of antibody was dissolved in 100 µl of 0.1 M PBS, 0.15 M NaCl and 10 mM EDTA buffer, pH 7.2 (final concentration 10 mg ml⁻¹), 0.6 mg of 2-mercaptoethylamine was added and the mixture was made to react for 90 minutes at 37 °C. The reduced antibody was purified by gel permeation. 3) Conjugation of the nanoparticles with the antibody: the nanoparticles activated with sulfo-SMCC and the reduced antibody were mixed in a ratio by weight of 4:1 (nanoparticles:antibody) in 0.1 M PBS, 0.15 M NaCl and 10 mM EDTA buffer, pH 7.2, and the mixture was made to react overnight at 4 °C. Finally, the doped nanoparticles conjugated with the antibody were collected by centrifugation and washed several times with Milli-Q water.

### Example 6 (Figure 4B) - conjugation by activation of the nanoparticles with sulfo-SMCC and activation of the antibody with 2-iminothiolane.

This method is divided into pre-activation of the doped nanoparticles, pre-activation of the antibody and conjugation in three separate reactions. 1) Activation of doped nanoparticles: the procedure is the same as described in Example 5. 2) Activation of the antibody: 1 mg of antibody was dissolved in 100 µl of 0.1 M PBS, 0.15 M NaCl and 10 mM EDTA buffer, pH 7.2 (final concentration 10 mg ml⁻¹), 0.5 mg of 2-iminothiolane was added and the mixture was made to react for 30 minutes at 37 °C. The activated antibody was purified by gel permeation. 3) Conjugation of the nanoparticles with the antibody: the doped nanoparticles activated with sulfo-SMCC and the antibody activated with 2-iminothiolane were mixed in a ratio by weight of 4:1 (nanoparticles:antibody) in 0.1 M PBS, 0.15 M NaCl and 10 mM EDTA buffer, pH 7.2, and the mixture was made to react overnight at 4 °C. Finally, the nanoparticles conjugated with the antibody were collected by centrifugation and washed several times with Milli-Q water.

### Example 7 (Figura 4C) - conjugation by activation of the nanoparticles with sulfo-SMCC and activation of the antibody with S-acetylthioglycolic ester N-hydroxysuccinimidic acid.

This method is divided into pre-activation of the doped nanoparticles, pre-activation of the antibody and conjugation in three separate reactions. 1) Activation of doped nanoparticles: the procedure is the same as described in Example 5. 2) Activation of the antibody: 1 mg of antibody was dissolved in 200 µl of 0.1 M PBS and 0.15 M NaCl buffer, pH 7.2 (final concentration 5 mg ml⁻¹), 40 µl of a solution of 8 mg ml⁻¹ SATA in DMF was added and the mixture was made to react for 30 minutes at room temperature. The sulfhydryl groups acetylated on the SATA-modified antibody were deprotected with hydroxylamine in excess by reacting for 2 hours at room temperature. The activated antibody was purified by gel permeation. 3) Conjugation of the nanoparticles with the antibody: the doped nanoparticles activated with sulfo-SMCC and the antibody activated with sulfhydryl groups were mixed in a ratio by weight of 4:1 (nanoparticles:antibody) in 0.1 M PBS, 0.15 M NaCl and 10 mM EDTA buffer, pH 7.2, and the mixture was made to react overnight at 4 °C. Finally, the nanoparticles conjugated with the antibody were collected by centrifugation and washed several times with Milli-Q water.

### Industrial applications

The present invention is applicable in all bioanalytical methods which require the determination of molecular targets with high sensitivity using biospecific probes marked with tracers. It provides thermochemiluminescent silica nanoparticles endowed with high detectability which can be employed for marking biospecific probes used in bioanalytical methods based on thermochemiluminescence measurements. The use of the doped silica nanoparticles of the invention as markers enables high performances to be achieved in assays.

In particular, the invention is applied to immunometric methods (based on the use of antibodies), methods based on nucleic acid hybridization (based on the use of oligonucleotides), methods based on natural receptors (e.g. proteins or receptors), methods based on synthetic receptors (e.g. aptamers consisting of peptides or synthetic nucleic acid analogues such as PNA, etc.).

The analytical formats for the above-described methods include single-tube analyses, microplate analyses, microarrays, immunohistochemical and immunocytochemical methods, methods of hybridization *in situ,* flow cytometry, microfluidic-based analytical systems, lateral flow immunoassay, etc.

In order to demonstrate the usability of silica nanoparticles as TCL markers in bioanalytical methods, two model assays were carried out, one based on immunological reactions (determination of ovalbumin) and one based on nucleic acid hybridization reactions (determination of Parvovirus B19 DNA),.

The following materials were used: bovine serum albumin (BSA), ovalbumin, 3-glycidoxypropyltrimethoxysilane (GOPS), mouse and rabbit anti-ovalbumin antibodies, mouse anti-digoxigenin antibody, rabbit anti-mouse antibody, buffers for hybridization reactions (hybridization buffer, denaturing buffer, washing buffer), PBS buffer (0.1 M phosphate buffer, pH = 7.4). The rabbit anti-mouse antibody marked with the silica nanospheres containing the dioxetane of formula (I) with R₁ = R₂ = R₃ = R₄ = Me was prepared according to the procedure which employs conjugation with N-hydroxysulfosuccinimide (see Example 1).

### Example 1. Determination of ovalbumin.

A microscope slide (borosilicate glass) was cleaned beforehand by treating it with a "piranha" solution (concentrated H₂SO₄ and 30% H₂O₂, 3:1 v/v) for 30 minutes, washing it with 90% ethanol, followed by 30% ethanol and distilled water, and then dried at 65 °C for 15 minutes. The slide was then treated with GOPS (10% v/v in toluene) for 2 hours at room temperature, washed twice in toluene, sonicated in toluene for 10 minutes and dried at 80 °C for 30 min. Spots of rabbit anti-ovalbumin antibody (0.1 µg/spot dissolved in PBS) were deposited on the slide using an automated spotter (VP 470 Slide Indexing System, Bio Gene, United Kingdom). The slide was incubated for 1 hour at room temperature in a wet chamber, then washed with PBS (3 x 5 min) and treated with a blocking solution (3% BSA in PBS) for two hours under stirring at room temperature. 100 µL of a solution of ovalbumin in PBS was then added, followed by another 1-hour incubation at room temperature in a wet chamber. After washing with PBS, 100 µL of solution of mouse anti-ovalbumin antibody dissolved in PBS was added to the slide, which was incubated for 1 hour at room temperature in a wet chamber. The slide was washed again in PBS and 100 µL of a solution of rabbit anti-mouse antibody marked with the silica nanoparticles containing the dioxetane of formula (I) with R₁ = R₂ = R₃ = R₄ = Me was added. After an additional hour of incubation in a wet chamber, the slide was washed with PBS and allowed to air dry. Then the thermochemiluminescence signal of the nanoparticles at the spots was measured by luminescence imaging using an LB-980 Night Owl luminograph (Berthold Technologies). The measurements were made by heating the slide using a flat heating element at a temperature of 70-80 °C.

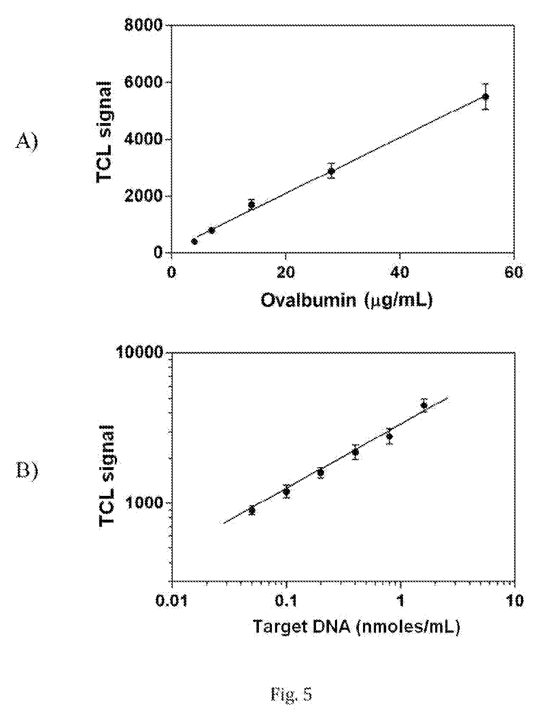

Figure 5A shows an example of a calibration curve of obtained from the analyses of standard solutions at different ovalbumin concentrations, from which it is possible to deduce a detection limit of about 90 ng/mL of ovalbumin.

### Example 2. Determination of Parvovirus B19 DNA.

A microscope slide was cleaned as previously described. Spots of capture probes specific for the target DNA sequence and modified at the 5' end with an NH₂group were denatured by heating for 5 minutes at 95 °C and deposited on the slide (0.1 pmol/spot) using an automated spotter (VP 470 Slide Indexing System, Bio Gene, United Kingdom). The slide was incubated for 1 hour at room temperature in a wet chamber, then washed with PBS (3 x 5 min) and treated with a blocking solution (3% BSA in PBS) for 1 hour under stirring at room temperature. 100 µL of a solution of the target DNA sequence obtained from a previous amplification process and marked with the hapten digoxigenin, treated with the denaturing buffer and then diluted in the hybridization buffer was added to the slide. After washing in the washing buffer (5 minutes), 100 µL of mouse anti-digoxigenin antibody was added to the slide, which was incubated for 1 hour at room temperature in a wet chamber. The slide was washed again in PBS and 100 µL of a solution of rabbit anti-mouse antibody marked with the silica nanoparticles containing the dioxetane of formula (I) with R₁ = R₂ = R₃ = R₄ = Me was added. After an additional hour of incubation in a wet chamber, the slide was washed with PBS and allowed to air dry. Then the thermochemiluminescence signal of the nanoparticles at the spots was measured by luminescence imaging using an LB-980 Night Owl luminograph (Berthold Technologies). The measurements were made by heating the slide using a flat heating element at a temperature of 70-80 °C.

Figure 5B shows an example of a calibration curve of obtained from the analyses of standard solutions at different concentrations of the target DNA sequence, from which it is possible to deduce a detection limit of about 50 pmol/mL of DNA.

## Claims

1. A thermochemiluminescent silica nanoparticle comprising a plurality of molecules of 1,2-dioxetane 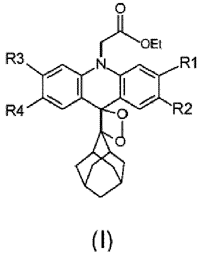 having the general formula (I):
wherein R₁, R₂, R₃ and R₄, are identical to each other or different from each other, and wherein R₁, R₂, R₃ and R₄ are selected from the group consisting of: -Me, -Et, -Pr, -iPr, -OMe, -OEt, -F, -Cl, -NH₂, - OH, -NO₂; or wherein R₁ and R₂, together with the carbon atom to which they are bonded, form an aromatic ring of 6 carbon atoms, and/or wherein R₂, and R₄, together with the carbon atom to which they are bonded, form an aromatic ring of 6 carbon atoms.

2. A thermochemiluminescent silica nanoparticle comprising a plurality of molecules of 1,2-dioxetane having the general formula (II):

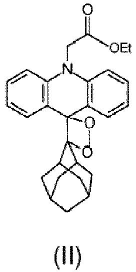

3. The nanoparticle according to claim 1 or 2, wherein said plurality of molecules of formula (I) or (II) is homogeneously dispersed in a silica matrix.

4. The nanoparticle according to any one of claims 1 to 3, further comprising fluorescent molecules capable of increasing the emission efficiency of the thermochemiluminescent reaction and/or of modifying the emission wavelength via a process of energy transfer from the excited products obtained from the decomposition of the thermochemiluminescent molecule, preferably wherein said fluorescent molecules are selected from the group consisting of dipyridamole, fluorescein, rhodamine and aromatic polycyclic hydrocarbons.

5. The nanoparticle according to any one of claims 1 to 4, derivatized on their surface with a large number of functional groups that enable the conjugation thereof to biospecific probes through the formation of covalent bonds.

6. The nanoparticle according to any one of claims 1 to 5, comprising from 1,000 to 10,000 molecules of 1,2-dioxetane of formula (I) and/or (II).

7. A method for synthesizing the nanoparticle according to any one of claims 1 to 6, which comprises the following steps: (a) formation of a microemulsion of oil in water, (b) addition of the thermochemiluminescent molecules and optionally fluorescent molecules according to claim 4; (c) addition of a source of silicon and a co-monomer responsible for the formation of the amino groups on the outer surface of the nanoparticles.

8. The method according to claim 7, which envisages forming the microemulsion of oil in water by mixing the latter with an oil, preferably n-butanol, and one or more surfactants, preferably AOT or Triton X-100.

9. The method according to claim 7 or 8, which envisages using triethoxyvinylsilane (TEOVS) as the source of silicon and 3-aminopropyitriethoxysilane (APTES) as the co-monomer.

10. A use of the nanoparticle according to any one of claims 1 to 6 as a marker of biospecific probes to be used in *in vitro* bioanalytical methods with detection by means of thermochemiluminescence measurements.

11. The use according to claim 10, wherein said biospecific probes are selected from the group consisting of: proteins, antibodies, receptors, oligonucleotides, and aptamers consisting of peptides or synthetic nucleic acid analogues, preferably PNA.

12. The use according to claim 11 for marking antibodies, comprising the following steps (a) activation of the nanoparticle and/or antibody, and (b) reaction between the nanoparticle and the antibody.

13. A use of antibodies marked with the nanoparticle according to any one of claims 1 to 6 in *in vitro* bioanalytical methods selected from the group consisting of : immunometric, immunocytochemical and immunohistochemical assays, lateral flow immunoassays, microfluidic analytic systems, flow cytometry and microarrays, with detection by means of thermochemiluminescence measurements.

## Patentansprüche

1. Thermochemilumineszente Silizium-Nanopartikel umfassend eine Vielzahl von 1,2-Dioxetanmolekülen 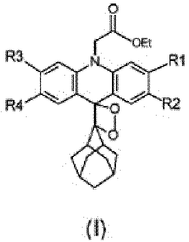 aufweisend die allgemeine Formel (I):
wobei R₁, R₂, R₃ und R₄, einander identisch oder voneinander unterschiedlich sind, und wobei R₁, R₂, R₃ und R₄ ausgewählt sind aus der Gruppe bestehend aus : - Me, -Et, -Pr, -iPr, -OMe, -OEt, -F, -Cl, -NH₂,- OH,-N0₂; oder wobei R₁ und R₂, zusammen mit dem Kohlenstoffatom, mit dem sie verbunden sind, einen aromatischen Ring von 6 Kohlenstoffatomen bilden, bzw.
wobei R₂, und R₄, zusammen mit dem Kohlenstoffatom, mit dem sie verbunden sind, einen aromatischen Ring von 6 Kohlenstoffatomen bilden.

2. Thermochemilumineszente Silizium-Nanopartikel umfassend eine Vielzahl von 1,2-Dioxetanmolekülen aufweisend die allgemeine Formel (II):

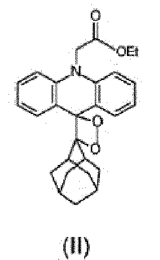

3. Nanopartikel nach Anspruch 1 oder 2, wobei genannte Vielzahl von Molekühlen der Formel (I) oder (II) in einer Siliziummatrix homogen dispergiert ist.

4. Nanopartikel nach einem der Ansprüche 1 bis 3, außerdem umfassend fluoreszente Moleküle, die in der Lage sind, die Emissionseffizienz der thermochemilumineszenten Reaktion zu steigern bzw. die Emissionswellenlänge über einen Prozess der Energieübertragung von den durch den Abbau des thermochemilumineszenten Moleküls erhaltenen angeregten Produkten zu modifizieren, wobei vorzugsweise genannte fluoreszente Moleküle ausgewählt sind aus der Gruppe bestehend aus Dipyridamol, Fluoreszein, Rhodamin und aromatischen polyzyklischen Kohlenwasserstoffen.

5. Nanopartikel nach einem der Ansprüche von 1 bis 4, derivatisiert auf ihrer Oberfläche mit einer großen Anzahl Funktionsgruppen, welche die Konjugation deren mit biospezifischen Proben durch die Bildung von kovalenten Bindungen ermöglicht.

6. Nanopartikel nach einem der Ansprüche 1 bis 5, umfassend von 1,000 bis 10,000 1,2-Dioxetanmolekülen der Formel (I) bzw. (II).

7. Methode zum Synthetisieren der Nanopartikel nach einem der Ansprüche 1 bis 6, was die folgenden Schritte umfasst: (a) Bildung einer Mikroemulsion von Öl in Wasser, (b) Dazugeben von thermochemilumineszenten Molekülen und optional fluoreszenten Molekülen nach Anspruch 4; (c) Dazugeben von einer Siliziumquelle und einem Comonomer, verantwortlich für die Bildung von Aminogruppen auf der Außenfläche der Nanopartikeln.

8. Methode nach Anspruch 7, welche die Mikroemulsion von Öl in Wasser vorsieht, durch Mischen des letztgenannten mit einem Öl, vorzugsweise n-Butanol, und einem oder mehreren Tensiden, vorzugsweise AOT oder Triton X-100.

9. Methode nach Anspruch 7 oder 8, welche die Verwendung von Triethoxyvinylsilan (TEOVS) als die Quelle von Silizium und 3-Aminopropyitriethoxysilan (APTES) als der Comonomer vorsieht.

10. Verwendung der Nanopartikel nach einem der Ansprüche 1 bis 6 als ein Marker von biospezifischen Proben, zum Einsatz kommend bei in vitro bioanalytischen Methoden mit Nachweis mittels Thermochemilumineszenz-Messungen.

11. Verwendung nach Anspruch 10, wobei genannte biospezifische Proben ausgewählt sind aus der Gruppe bestehend aus: Proteinen, Antikörpern, Rezeptoren, Oligonukleotiden, und Aptameren bestehend aus Peptiden oder synthetischen Nukleinsäure-Analoga, vorzugsweise PNA.

12. Verwendung nach Anspruch 11 zur Markierung von Antikörpern, umfassend die folgenden Schritte (a) Aktivierung der Nanopartikel bzw. des Antikörpers, und (b) Reaktion zwischen der Nanopartikel und dem Antikörper.

13. Verwendung von Antikörpern markiert mit der Nanopartikel nach einem der Ansprüche 1 bis 6 bei in vitro bioanalytischen Methoden ausgewählt aus der Gruppe bestehend aus: immunometrischen, immunozytochemischen und immunohistochemischen Assays, Lateral flow Immunoassays, mikrofluidischen Analysesystemen, Durchflusszytometrie und Mikroarrays, mit Nachweis mittels Thermochemilumineszenz-Messungen.

## Revendications

1. Nanoparticule de silice thermochimioluminescente comprenant une pluralité de molécules de 1,2-dioxétane 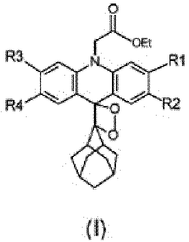 ayant la formule générale (I) :
dans laquelle R₁, R₂, R₃ et R₄ sont identiques les uns aux autres ou différents les uns des autres, et dans laquelle R₁, R₂, R₃ et R₄ sont choisis dans le groupe constitué par le : -Me, -Et, -Pr, -iPr, -OMe, -OEt, -F, -Cl, -NH₂, - OH, -NO₂ ; ou dans laquelle R₁ et R₂, avec l'atome de carbone auquel ils sont liés, forment un noyau aromatique de 6 atomes de carbone, et/ou dans laquelle R₂ et R₄, avec l'atome de carbone auquel ils sont liés, forment un noyau aromatique de 6 atomes de carbone.

2. Nanoparticule de silice thermochimioluminescente comprenant une pluralité de molécules de 1,2-dioxétane ayant la formule générale (II) :

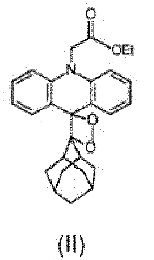

3. Nanoparticule selon la revendication 1 ou 2, dans laquelle ladite pluralité de molécules de formule (I) ou (II) est dispersée de façon homogène dans une matrice de silice.

4. Nanoparticule selon l'une quelconque des revendications de 1 à 3, comprenant de plus des molécules fluorescentes capable d'augmenter l'efficacité d'émission de la réaction thermochimioluminescente et/ou de modifier la longueur d'onde d'émission via un processus de transfert d'énergie à partir des produits excités obtenus à partir de la décomposition de la molécule thermochimioluminescente, de préférence dans laquelle lesdites molécules fluorescentes sont choisies dans le groupe constitué par le dipyridamole, la fluorescéine, la rhodamine et les hydrocarbures polycycliques aromatiques.

5. Nanoparticule selon l'une quelconque des revendications de 1 à 4, dérivée sur sa surface avec un grand nombre de groupes fonctionnels permettant sa conjugaison à des sondes biospécifiques à travers la formation de liaisons covalentes.

6. Nanoparticule selon l'une quelconque des revendications de 1 à 5, comprenant de 1 000 à 10 000 molécules de 1,2-dioxétane de formule (I) et/ou (II).

7. Procédé de synthétisation d'une nanoparticule selon l'une quelconque des revendications de 1 à 6, comprenant les étapes suivantes : (a) formation d'une microémulsion d'huile dans de l'eau, (b) ajout des molécules thermochimioluminescentes et éventuellement des molécules fluorescentes selon la revendication 4 ; (c) ajout d'une source de silicium et d'un comonomère responsable de la formation des groupes amino sur la surface extérieure des nanoparticules.

8. Procédé selon la revendication 7, qui prévoit la formation de la microémulsion d'huile dans de l'eau en mélangeant cette dernière à une huile, de préférence du n-butanol, et à un ou plusieurs surfactants, de préférence de l'AOT ou du Triton X-100.

9. Procédé selon la revendication 7 ou 8, qui prévoit d'utiliser du triéthoxyvinylsilane (TEOVS) comme étant la source de silicium et du 3-aminopropyitriéthoxysilane (APTES) comme comonomère.

10. Utilisation de la nanoparticule selon l'une quelconque des revendications de 1 à 6 comme un marqueur de sondes biospécifiques à utiliser dans des procédés bioanalytiques in vitro par détection au moyen de mesures de thermochimioluminescence.

11. Utilisation selon la revendication 10, dans laquelle lesdites sondes biospécifiques sont choisies dans un groupe constitué par les : protéines, anticorps, récepteurs, oligonucléotides et des aptamères consistant en des peptides ou des analogues d'acide nucléique synthétique, de préférence l'APN.

12. Utilisation selon la revendication 11 servant à marquer des anticorps, comprenant les étapes suivantes (a) activation de la nanoparticule et/ou de l'anticorps, et (b) réaction entre la nanoparticule et l'anticorps.

13. Utilisation d'anticorps marqués avec la nanoparticule selon l'une quelconque des revendications de 1 à 6 dans des procédés bioanalytiques in vitro choisis dans le groupe constitué par des : tests immunométriques, immunocytochimiques et immunihistochimique, tests d'immunochromatographie sur membrane, systèmes analytiques à micro-fluides, cytométrie en flux et puces à ADN, avec détection par le biais de mesures de thermochimioluminescence.
